**Europäisches Patentamt**

(19) **European Patent Office**

**Office européen des brevets**

(11) Publication number : **0 332 444 B1**

# EUROPEAN PATENT SPECIFICATION

(12)

(45) Date of publication of patent specification :
**01.07.92 Bulletin 92/27**

(51) Int. Cl.⁵ : **G01N 33/68,** G01N 33/50

(21) Application number : **89302351.5**

(22) Date of filing : **09.03.89**

(54) **Reagent for diagnosis of cancer.**

(30) Priority : **11.03.88 KR 251188**

(43) Date of publication of application :
**13.09.89 Bulletin 89/37**

(45) Publication of the grant of the patent :
**01.07.92 Bulletin 92/27**

(84) Designated Contracting States :
**DE FR GB IT SE**

(56) References cited :
**EP-A- 0 103 189
US-A- 4 021 198**

(73) Proprietor : **SAM I1 PHARMACEUTICAL
MANUFACTURING CO., Ltd
990-1, Bangbae-1 Dong
Seocho-ku Seoul (KR)**

(72) Inventor : **Kim, Young Chin
Hyundae Apt. 78-104 Apkujung-Dong
Kangam-Ku
Seoul (KR)**

(74) Representative : **Pendlebury, Anthony et al
PAGE, WHITE & FARRER 54 Doughty Street
London WC1N 2LS (GB)**

EP 0 332 444 B1

Jouve, 18, rue Saint-Denis, 75001 PARIS

## Description

The present invention relates to a mixed Hg-Ni reagent for diagnosing cancers by detection of aromatic amines, such as tyrosine, commonly present in the urine of a cancer patient.

A prior technique which measures the quantity of aromatic amines (tyrosine, etc), which are detected specifically in the urine of cancer patients, for the diagnosis of cancer is described in Korean Patent No. 21558 in the name of the present inventor. An unclear magnetic resonance signal in the range of 3.00 ppm to 3.09 ppm is commonly observed in the urine of cancer patients, due to the presence of aromatic amines. A diagnostic method using Millon's reagent for detection of cancers has already been disclosed in the prior patent.

However, the use of the Millon's reagent as a reagent for confirming the presence of tyrosine, in the urine test has a disadvantage. That is, the precipitate formed in the reaction is very unstable. The reason for that is that mercury ions in Millon's reagent have a high complex-forming capacity but a relatively low ionization tendency in comparison with other metal ions. Thus, it is easily interfered with by inorganic salts and aromatic organo-compounds which are present in the urine sample.

Accordingly, in order to overcome this disadvantage we tried to react tyrosine standard material with other metallic ions in the place of the mercury ions, based on the fact that the reaction between Millon's reagent and tyrosine is primarily due to the formation of a complex by the said mercury ions. As a result, we have confirmed that tyrosine also reacts with nitric acid solutions of cadmium (Cd), zinc (Zn), copper (Cu) and nickel (Ni) to form a red complex compound. However, when the nitric acid solutions of these metals are applied to the urine samples of cancer patients in place of Millon's reagent, no reaction is observed. The reason for this appears to be that the complex-forming capacity of these metal ions is relatively lower than that of the mercury ion, and the content of aromatic amines present in the urine of a cancer patient is so small that it does not reach the concentration level capable of initiating the reaction with the metal ions. However, these metals have a low complex-forming capacity, and a high ionization tendency. Accordingly, it is supposed that these metals may function as either a substituent for mercury in the mercury complexes or a cross-linking agent between the complexes to stabilize the complexes. On the basis of this, Millon's reagent, which is a nitric acid solution of mercury was combined with each of the above mentioned metals and it was confirmed that the combination of Millon's reagent with nickel gave more stable precipitate than Millon's reagent alone.

That is, when Millon's reagent is used alone, the complex obtained is unstable and dissolved easily depending on the condition of the sample, and therefore, the reading of the results may be difficult.

However, the combined reagent of the present invention does not produce such an undesired phenomenon and the reading of the reaction results is easy because the color of the reaction precipitate is stable and persists for a long period.

According to the present invention there is provided a cancer diagnostic reagent composition comprising mercury, nickel, nitric acid and distilled water, wherein nickel is contained in an amount of 0.05 to 0.5 parts by weight per one part by weight of mercury.

The composition of the present invention consists of mercury, nickel, nitric acid and distilled water. In this composition, if the mixing ratio between mercury and nickel is higher in mercury, the precipitate obtained is unstable, and if the ratio is higher in nickel, the amount of the precipitate is reduced bringing about a poor diagnostic effect. Therefore, it is necessary to maintain a suitable mixing ratio. In general, the suitable amount of nickel needed for maintaining the stable color of the precipitate during the reading period is 0.1 to 0.3 part by weight of nickel per one part by weight of mercury. In addition, nitric acid included in the reagent composition of the present invention is generally used in the ratio of 2 to 5 ml of nitric acid per gram of mercury. Distilled water is used in a substantially similar amount to nitric acid. However, if distilled water is used in excess, the amount of precipitate is reduced while too small amount of distilled water will result an unstable precipitate.

Further referred embodiments are mentioned in claims 3 and 4.

The following Examples illustrate the present invention.

### Example 1

10 ml of a solution of 50 g of metallic mercury dissolved in 50 ml of concentrated nitric acid is mixed with 20 ml of a solution of 5 g of metallic nickel dissolved in 50 ml of concentrated nitric acid. The mixture is diluted with 30 ml of distilled water.

When 0.15 ml of the reagent of the present invention, which is composed in the above ratio, is added to 3 ml of the urine samples, a red precipitate is observed in the urine of cancer patients while a white precipitate is observed in the urine of healthy people.

The clinical tests according to the above method are practiced for 37 healthy people, 62 cancer patients and 86 people suffering from other diseases. The results in the following table are obtained (Table 1). The clinical tests using the known Millon's reagent are practiced for the same samples, and as can be seen in the following table, the reliability of the test results thereof is low and is more decreased with the passage of the test time (Table 2).

## Table 1.
### Clinical data using the reagent of the present invention

| Subject Patient Group | Positive(%) | Negative | Total |
|---|---|---|---|
| Healthy control group | 3(8.1) | 34 | 37 |
| Other disease group | 18(14.9) | 103 | 121 |
| Cancer patient group | 49(79.0) | 13 | 62 |

$$\text{Sensitivity} = \frac{49}{49 + 13} = 79.0\%$$

$$\text{Specificity} = \frac{137}{137 + 21} = 86.7\%$$

$$\text{Positive reliability} = \frac{49}{49 + 21} = 70.0\%$$

$$\text{Negative reliability} = \frac{137}{137 + 13} = 91.3\%$$

## Table 2.
### Comparative Clinical data using the known Millon's reagent

| Subject Patient Group | Positive(%) | Negative | Total |
|---|---|---|---|
| Healthy control group | 8(21.6) | 29 | 37 |
| Other disease group | 32(26.5) | 89 | 121 |
| Cancer patient group | 38(61.3) | 24 | 62 |

$$\text{Sensitivity} = \frac{38}{38 + 24} = 63.3\%$$

$$\text{Specificity} = \frac{118}{118 + 40} = 74.7\%$$

$$\text{Positive reliability} = \frac{38}{38 + 40} = 48.7\%$$

$$\text{Negative reliability} = \frac{118}{118 + 24} = 83.1\%$$

As can be apparently confirmed from the above test results, the reagent composition of the present invention, which is superior to the known Millon's reagent, can be used as an excellent cancer diagnostic reagent composition.

## Claims

1. A cancer diagnostic reagent composition comprising mercury, nickel, nitric acid and distilled water, wherein nickel is contained in an amount of 0.05 to 0.5 parts by weight per one part by weight of mercury.

2. A reagent composition according to claim 1, wherein the amount of nickel is 0.1 to 0.3 parts by weight per one part by weight of mercury.

3. A reagent composition according to claim 1 or 2, wherein 0.5 to 2 parts by volume of distilled water is used per one part by volume of nitric acid.

4. A reagent composition according to claim 1 or 2, wherein the weight ratio between mercury and nickel is 1 : 0.2 and the volume ratio between nitric acid and distilled water is 3 : 2 to 4.

5. A process for preparing a cancer diagnostic reagent composition according to claim 1 which comprises adding distilled water to a solution of mercury in concentrated nitric acid and a solution of nickel in concentrated nitric acid.

## Patentansprüche

1. Krebsdiagnostische Reagenzmischung aus Quecksilber, Nickel, Salpetersäure und destilliertem Wasser, worin Nickel in einer Menge von 0,05 bis 0,5 Gewichtsteilen pro Gewichtsteil Quecksilber enthalten ist.

2. Reagenzmischung nach Anspruch 1, worin die Menge an Nickel 0,1 bis 0,3 Gewichtsteile pro Gewichtsteil Quecksilber beträgt.

3. Reagenzmischung nach Anspruch 1 oder 2, worin 0,5 bis 2 Volumenteile destilliertes Wasser pro Volumenteil Salpetersäure eingesetzt werden.

4. Reagenzmischung nach Anspruch 1 oder 2, worin das Gewichtsverhältnis zwischen Quecksilber und Nickel 1 : 0,2 und das Volumenverhältnis zwischen Salpetersäure und destilliertem Wasser 3 : 2 bis 4 beträgt.

5. Verfahren zur Herstellung einer Krebsdiagnostischen Reagenzmischung nach Anspruch 1, wobei man destilliertes Wasser zu einer Lösung von Quecksilber in konzentrierter Salpetersäure und einer Lösung von Nickel in konzentrierter Salpetersäure gibt.

## Revendications

1. Composition de réactif pour le diagnostic du cancer, comprenant du mercure, du nickel, de l'acide nitrique et de l'eau distillée, dans laquelle le nickel est contenu en une quantité de 0,05 à 0,5 parties en poids pour une partie en poids de mercure.

2. Composition de réactif suivant la revendication 1, dans laquelle la quantité de nickel est de 0,1 à 0,3 parties en poids pour une partie en poids de mercure.

3. Composition de réactif suivant la revendication 1 ou 2, dans laquelle 0,5 à 2 parties en volume d'eau distillée sont utilisées pour une partie en volume d'acide nitrique.

4. Composition de réactif suivant la revendication 1 ou 2, dans laquelle le rapport de poids entre le mercure et le nickel est de 1 : 0,2 et le rapport de volume entre l'acide nitrique et l'eau distillée est de 3 : 2 à 4.

5. Procédé pour préparer une composition de réactif pour le diagnostic du cancer suivant la revendication 1, comprenant l'addition d'eau distillée à une solution de mercure dans l'acide nitrique concentré et à une solution de nickel dans l'acide nitrique concentré.